# FASCICULE DE BREVET EUROPEEN

(11) **EP 0 715 615 B1**
(45) Date de publication et mention de la délivrance du brevet: **07.10.1998**
(21) Numéro de dépôt: 95921090.7
(22) Date de dépôt: 21.06.1995
(51) Int. Cl.: C07C 59/205, A61K 7/46, C07C 59/11, C07C 59/82, C07C 69/716, C07C 69/738

(54) **PROCEDE POUR LA PREPARATION DE L'ACIDE (+)-(1R)-CIS-3-OXO-2-PENTYL-1-CYCLOPENTANEACETIQUE**
VERFAHREN ZUR HERSTELLUNG VON (+)-(1R)-CIS-3-OXO-2-PENTYL-1-CYCLOPENTANESSIGSÄURE
METHOD FOR PREPARING (+)-(1R)-CIS-3-OXO-2-PENTYL-1-CYCLOPENTANEACETIC ACID

(30) Priorité: 23.06.1994 CH 2006/94
(43) Date de publication de la demande: 12.06.1996
(73) Titulaire: FIRMENICH SA, 1211 Genève 8 (CH)
(72) Inventeur: RAUTENSTRAUCH, Valentin, CH-1233 Bernex (CH); RIEDHAUSER, Jean-Jacques, CH-1258 Certoux (CH)
(74) Mandataire: Salvaterra-Garcia, Maria de Lurdes
(86) Numéro de dépôt international: IB9500505
(87) Numéro de publication internationale: WO9600206

(56) Documents cités:
- NL-A- 6 918 228
- US-A- 5 300 489
- JOURNAL OF THE AMERICAN CHEMICAL SOCIETY, vol.113, no.22, 23 Octobre 1991, WASHINGTON, DC US pages 8518 - 8519 M.J.BURK 'C2-SYMMETRIC BIS(PHOSPHOLANES) AND THEIR USE IN HIGHLY ENANTIOSELECTIVE HYDROGENATION REACTIONS.' cité dans la demande

## Description

La présente invention a trait au domaine de la synthèse organique et, plus particulièrement, à celui de la synthèse stéréosélective et énantiosélective de composés optiquement actifs. Elle concerne, en particulier, la préparation de l'acide (+)-(1R)-cis-3-oxo-2-pentyl-1-cyclopentaneacétique, ou acide (+)-(1R,2S)-3-oxo-2-pentyl-1-cyclopentaneacétique, dans un état optiquement pur, ainsi que de son ester méthylique.

L'acide (+)-(1R)-cis-3-oxo-2-pentyl-1-cyclopentaneacétique est un composé de structure connue. Il a en effet été décrit par O. Miersch et al. dans Phytochem. 26, 1037 (1987). Selon ces auteurs, l'acide susmentionné a été extrait d'une culture de *Botryodiplodia theobromae* isolés d'oranges d'origine cubaine. Cependant, à notre connaissance, il n'existe dans l'art antérieur aucune description d'une méthode de synthèse de cet acide susceptible d'être appliquée convenablement à l'échelle industrielle. Par ailleurs, on ne connaît pas la pureté optique du composé décrit.

Or, la présente invention apporte justement une solution à ce problème. Elle fournit l'acide susmentionné avec une pureté optique excellente et essentiellement sous la forme de son isomère de configuration (+)-(1R)-cis. Ceci est d'autant plus important que l'acide (+)-(1R)-cis-3-oxo-2-pentyl-1-cyclopentaneacétique est un produit utile pour la préparation du (+)-(1R)-cis-3-oxo-2-pentyl-1-cyclopentaneacétate de méthyle, l'isomère préféré, du point de vue olfactif, du dihydrojasmonate de méthyle, aussi connu sous le nom d'Hédione ® (origine: Firmenich SA, Genève, Suisse), un ingrédient très prisé et largement utilisé en parfumerie.

Parmi les quatre isomères optiquement actifs du dihydrojasmonate de méthyle, ou Hédione ®, il est en effet connu que le (+)-(1R)-cis-3-oxo-2-pentyl-1-cyclopentaneacétate de méthyle possède au mieux les caractères odorants, et notamment la note jasminée, recherchés dans l'Hédione®. Ainsi, l'obtention du (+)-(1R)-cis-3-oxo-2-pentyl-1-cyclopentaneacétate de méthyle optiquement pur revêt une importance capitale en parfumerie. Si l'on sait en plus qu'il n'existe à ce jour aucune synthèse de ce composé pouvant être exploitée à l'échelle industrielle, on comprend la valeur de la présente invention, laquelle permet d'obtenir industriellement un produit de départ dont la transformation à large échelle en le (+)-(1R)-cis-3-oxo-2-pentyl-1-cyclopentaneacétate de méthyle est aussi rentable.

On connaît du brevet CH-A-561 168 un procédé qui permet d'obtenir le cis-3-oxo-2-pentyl-1-cyclopentaneacétate de méthyle avec une pureté d'environ 90%, mais ce composé est un mélange racémique, dont le contenu en (+)-(1R)-cis-3-oxo-3-pentyl-1-cyclopentaneacétate de méthyle ne dépasse pas 45% en poids du mélange.

### Exposé de l'invention

Un premier objet de l'invention est donc un procédé pour la préparation de l'acide 3-oxo-2-pentyl-1-cyclopentaneacétique, ou du 3-oxo-2-pentyl-1-cyclopentaneacétate de méthyle, essentiellement sous forme de leur isomère de configuration (+)-(1R)-cis, caractérisé en ce qu'il comprend les étapes suivantes:
a) l'hydrogénation catalytique, dans un solvant approprié et en présence d'un catalyseur constitué par un complexe de Ru (II) comportant des ligands chiraux qui contiennent un radical de type 2,2'-bis(diphénylphosphino)-1,1'-binaphtyle (BINAP) ou de type 1,2-bis(2,5-dialkylphospholano) benzényle (DuPHOS), l'alkyle étant un radical de C₂ ou C₃, d'un composé de formule 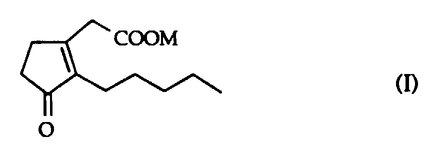 dans laquelle le symbole M représente un atome d'hydrogène, un atome d'un métal alcalin ou alcalino-terreux ou un groupe NR₄, R représentant l'hydrogène ou un radical alkyle inférieur, pour obtenir un composé de formule 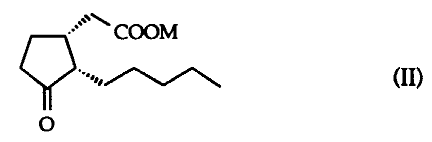 dans laquelle M a le sens indiqué ci-dessus, essentiellement sous forme de l'isomère de configuration (+)-(1R)-cis;
b) le cas échéant, l'acidification, de façon connue en soi, du composé de formule (II) dans laquelle M représente un atome d'un métal alcalin ou alcalino-terreux ou un groupe NR₄, R représentant l'hydrogène ou un radical alkyle inférieur, pour obtenir l'acide (+)-(1R)-cis-3-oxo-2-pentyl-1-cyclopentaneacétique; et
c) le cas échéant, l'estérification, dans des conditions appropriées, de l'acide (+)-(1R)-cis-3-oxo-2-pentyl-1-cyclopentaneacétique ainsi obtenu pour former le (+)-(1R)-cis-3-oxo-2-pentyl-1-cydopentaneacétate de méthyle.

Par métal alcalin ou alcalino-terreux, on entend ici n'importe lequel des éléments du groupe I, ou respectivement II, du Tableau Périodique de Mendelejev. A ce titre, on peut citer plus particulièrement le sodium, le potassium, le lithium, le césium, le calcium et le magnésium.

D'autre part, les radicaux alkyles inférieurs représentés par le symbole R incluent typiquement des radicaux alkyles linéaires ou ramifiés ayant de 1 à 3 atomes de carbone.

Les catalyseurs utilisés dans le procédé selon l'invention sont des complexes de ruthénium (II) dans lesquels ce métal est lié à des ligands chiraux du type BINAP, c'est-à-dire dérivés du 2,2'-bis(diphénylphosphino)-1,1-binaphtalène, ou de la famille des 1,2-bis(phospholano)benzènes ou DuPHOS. En ce qui concerne ces derniers, ils contiennent plus particulièrement des radicaux du type 1,2-bis(2,5-dialkylphospholano) benzényle, étant entendu ici par "alkyle" un radical contenant de 1 à 3 atomes de carbone. Des exemples particuliers de produits commerciaux sont le 1,2-bis(2,5-diméthylphospholano)benzényle, couramment désigné par Me-DuPHOS et le 1,2-bis(2,5-diéthylphospholano)benzényle ou Et-DuPHOS (voir, par exemple, M.J. Burk, J. Amer. Chem. Soc. 1991, 113, 8518).

Ce sont des composés optiquement actifs connus pour leur activité catalytique élevée et qui sont couramment utilisés dans des réactions d'hydrogénation sélective d'oléfines. Cependant, malgré la vaste littérature y relative, notamment en ce qui concerne ceux qui contiennent des ligands de type BINAP, nous n'avons pas décelé un seul exemple d'utilisation d'un tel catalyseur dans l'hydrogénation d'une double liaison cyclique et tétrasubstituée, c'est-à-dire dans laquelle tous les substituants sont différents de l'hydrogène, comme c'est le cas pour le procédé présent. C'est avec surprise qu'il a été constaté que l'hydrogénation susmentionnée fournissait un produit essentiellement de configuration cis, et ceci avec un rendement excellent, même dans le cas où le produit de départ est l'acide 3-oxo-2-pentyl-1-cyclopentène-1-acétique, malgré l'instabilité de ce dernier.

La réaction d'hydrogénation qui caractérise le procédé de l'invention est le résultat d'essais fort nombreux, essais effectués à l'aide de catalyseurs variés, par exemple à base de rhodium, dont par ailleurs la nature était parfois très proche de celle des complexes de Ru(II) employés à présent. Le succès obtenu avec ces derniers est d'autant plus surprenant que, malgré qu'il était connu que lesdits catalyseurs à base de rhodium favorisaient la réduction de fonctions analogues à celle notamment de l'acide de départ dans le procédé de l'invention, ils se sont montrés moins efficaces dans la réduction de l'acide 3-oxo-2-pentyl-1-cyclopentène-1-acétique, contrairement aux catalyseurs de ruthénium (II) utilisés selon l'invention.

Selon un mode d'exécution particulier de l'invention, le procédé est caractérisé en ce qu'on soumet l'acide 3-oxo-2-pentyl-1-cyclopentène-1-acétique à hydrogénation catalytique dans un solvant organique, en présence du bis(trifluoroacétato)[(+)-(R)-BINAP]ruthénium (II) ou du bis(trifluoroacétato) [(-)-(R,R)-Et-DuPHOS]ruthénium (II).

En tant que solvant organique, on peut utiliser un solvant d'usage courant dans ce type de réactions, par exemple un hydrocarbure chloré, un alcool ou autre. De meilleurs rendements ont été obtenus lorsque l'on a utilisé un mélange de dichlorométhane et méthanol ou de tétrahydrofurane et méthanol.

Par ailleurs, il a aussi été observé qu'il était préférable d'effectuer la réaction en présence d'une base organique azotée, par exemple une trialkylamine ou une pyridine. La concentration dans laquelle cette base peut être ajoutée au milieu réactionnel varie dans un rapport molaire de 1 : 1 à 4:1, par rapport au catalyseur de Ru (II). De très bons rendements en produit final ont été obtenus lorsque l'on a ajouté la triéthylamine dans un rapport molaire compris entre 1,5:1 et 4:1.

Selon un mode d'exécution particulier du procédé de l'invention, les catalyseurs de Ru (II) susmentionnés sont préparés in situ, à partir de produits d'origine commerciale et selon des méthodes connues en soi [voir, par exemple, B. Heiser et al., Tetrahedron : Asymmetry 1991, 2, pp. 43 et 51].

L'acide 3-oxo-2-pentyl-1-cyclopentène-1-acétique de départ est préparé par hydrolyse du 3-oxo-2-pentyl-1-cyclopentène-1-acétate de méthyle [décrit dans US 5,302,745 ou EP 583 564] dans les conditions décrites plus loin.

L'acide (+)-(1R)-cis-3-oxo-2-pentyl-1-cyclopentaneacétique ainsi obtenu peut ensuite être utilisé selon l'invention pour préparer le (+)-(1R)-cis-3-oxo-2-pentyl-1-cydopentaneacétate de méthyle, par exemple par traitement avec un excès de diazométhane en solution éthérée.

Bien entendu, lorsque l'hydrogénation a lieu en présence de bis(trifluoroacétato)[(-)-(S)-2,2'-bis-(diphénylphosphino)-1,1'-binaphtyl] ruthénium (II) ou de bis(trifluoroacétato)[(+)-(S,S)-Et-DuPHOS]ruthénium (II), on obtient l'acide (-)-(1S)-cis-3-oxo-2-pentyl-1-cyclopentaneacétique, lequel peut être utilisé pour préparer le (-)-(1S)-cis-3-oxo-2-pentyl-1-cyclopentaneacétate de méthyle.

Un autre mode préférentiel d'exécution de l'invention est un procédé caractérisé en ce qu'on soumet à hydrogénation catalytique, dans un solvant approprié et en présence d'un catalyseur constitué par un complexe de ruthénium (II) et (+)-(R)-BINAP-sulfoné, un composé de formule (I) dans laquelle M représente un atome d'un métal alcalin ou alcalino-terreux ou un groupe NR₄, R représentant l'hydrogène ou un radical alkyle inférieur.

Le catalyseur susmentionné est un complexe de ruthénium (II) avec le (+)-(R)-2,2'-bis-(diphénylphosphino)-1,1'-binaphtyl sulfoné, ce ligand chiral étant obtenu par sulfonation du BINAP, de façon analogue à celle décrite par Kam-to Wan et al., J. Chem. Soc. Chem. Comm. 1993, 1262. Selon ces auteurs, le produit de cette sulfonation semble être majoritairement un BINAP tétrasulfoné, c'est-à-dire BINAP(SO₃Na)₄, les groupes sulfonate étant apparemment attachés aux substituants phényle du BINAP. Les conditions détaillées de la préparation de ce type de catalyseur sont décrites plus loin.

Parmi les composés de formule (I) susmentionnés, utilisés comme produits de départ dans ce mode d'exécution préférentiel du procédé de l'invention, on fera usage de préférence du 3-oxo-2-pentyl-1-cyclopentène-1-acétate de lithium.

Par ailleurs, ces composés de formule (I) dans laquelle M représente un atome d'un métal alcalin ou alcalino-terreux ou un groupe NR₄, R représentant l'hydrogène ou un radical alkyle inférieur, sont également des composés nouveaux faisant l'objet de l'invention. Ils sont préparés à partir de l'acide 3-oxo-2-pentyl-1-cyclopentaneacétique comme il est décrit plus loin.

En tant que solvant, on utilisera de préférence un solvant permettant d'obtenir un milieu de réaction homogène. A cet effet, on peut employer un solvant aqueux, notamment de l'eau ou un mélange d'eau et d'un alcool, tel que par exemple le méthanol. Les proportions relatives d'eau et d'alcool dans ces mélanges ne se sont pas révélées critiques.

Ce mode d'exécution du procédé de l'invention s'est révélé très avantageux, permettant une récupération plus aisée et efficace du catalyseur, ainsi que du produit de l'hydrogénation.

Le procédé qui fait l'objet de la présente invention peut par ailleurs, si désiré, comprendre des étapes supplémentaires permettant de purifier davantage les produits obtenus comme il a été décrit jusqu'ici et notamment d'améliorer le rapport isomérique cis/trans de ces produits dans les cas où cela pourrait s'avérer utile. Ainsi, l'invention fournit également un procédé tel que décrit auparavant, caractérisé en ce qu'il comporte en plus la purification de l'acide (+)-(1R)-cis-3-oxo-2-pentyl-1-cyclopentaneacétique ou du (+)-(1R)-cis-3-oxo-2-pentyl-1-cyclopentaneacétate de méthyle obtenus, laquelle purification comprend :
a) la réduction à l'aide de tri-(sec-butyl)-borohydrure de lithium de (+)-(1R)-cis-3-oxo-2-pentyl-1-cyclopentane-acétate de méthyle ;
b) le traitement du mélange réactionnel ainsi obtenu avec H₂O₂ et un excès de NaOH et l'acidification subséquente du sel résultant pour obtenir un mélange contenant essentiellement l'acide (+)-(1R,2S,3R)-3-hydroxy-2-pentyl-1-cyclopentaneacétique et l'acide (-)-(1R,2S,3S)-3-hydroxy-2-pentyl-1-cyclopentaneacétique ;
c) le traitement thermique dudit mélange dans le toluène, avec séparation de l'eau par distillation azéotropique, suivi d'un traitement avec carbonate de potassium pour isoler la (-)-(1R,8S)-8-pentyl-2-oxabicyclo [3.2.1]octan-3-one formée; et
d) le traitement de cette lactone avec NaOH, suivi d'acidification, pour obtenir l'acide (+)-(1R,2S,3R)-3-hydroxy-2-pentyl-1-cyclopentane-acétique pratiquement pur;
e) l'oxydation de cet acide à l'aide de chlorochromate de pyridinium, en présence d'acétate de sodium; et, le cas échéant,
f) l'estérification, dans des conditions appropriées, de l'acide (+)-(1R)-cis-3-oxo-2-pentyl-1-cyclopentaneacétique ainsi formé, pour obtenir le (+)-(1R)-cis-3-oxo-2-pentyl-1-cyclopentaneacétate de méthyle pratiquement pur.

Le procédé de purification susmentionné repose sur la découverte que, contrairement aux procédés classiques de réduction des composés tels que le 3-oxo-2-pentyl-1-cyclopentaneacétate de méthyle et analogues, lesquels procédés font usage par exemple de borohydrure de sodium, et lesquels conduisent à l'obtention de mélanges contenant tous les isomères de l'acide 3-hydroxy-2-pentyl-1-cyclopentaneacétique [voir, par exemple, E. Demole et al., Helv. Chim. Acta 45, 675 (1962)], l'utilisation selon l'invention du tri-(sec-butyl)-borohydrure de lithium ou L-Selectride ® (Aldrich) pour réduire le (+)-(1R)-cis-3-oxo-2-pentyl-1-cydopentane-acétate de méthyle contenant de faibles quantités d'isomères trans, se révèle être parfaitement sélective, ne fournissant que les composés dont la configuration est représentée ci-après (pour des espèces racémiques)

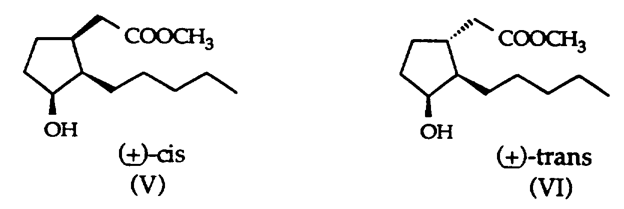

Ce résultat inattendu se révèle d'une importance capitale. En effet, il s'est avéré que lors de la saponification de ces esters à l'aide de NaOH, et de l'acidification subséquente des sels obtenus, laquelle engendre la formation des acides correspondants, seul l'acide de configuration entièrement cis, de formule 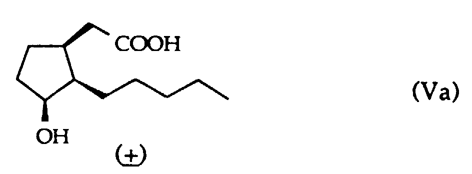 est à même de lactoniser lors d'un traitement thermique dans le toluène, avec élimination azéotropique de l'eau, l'acide correspondant à l'ester de formule (VI) restant inerte dans ces conditions et pouvant ainsi être séparé de la lactone formée par extraction avec carbonate de potassium. Cette lactone, à savoir la 8-pentyl-2-oxabicydo[3.2.1]octan-3-one, ou le cas échéant, l'un de ses isomères optiquement actifs, peut ensuite être reconvertie dans l'acide (Va) à l'aide de méthodes classiques. Cet acide de configuration entièrement cis permet ensuite d'obtenir l'acide cis-3-oxo-2-pentyl-1-cyclopentaneacétique ou, le cas échéant, l'un de ses énantiomères, à l'état pur, par simple oxydation à l'aide de chlorochromate de pyridinium (PCC).

Le procédé de l'invention décrit ci-dessus permet ainsi d'éliminer les isomères de configuration trans éventuellement présents dans n'importe lequel des produits obtenus comme décrit auparavant, à savoir par hydrogénation des composés (I), suivie éventuellement de l'acidification des composés (II) et d'estérification de l'acide 3-oxo-2-pentyl-1-cyclopentaneacétique de configuration essentiellement (1R)-cis formé. Cette méthode de purification complémentaire se révèle particulièrement utile lorsque les produits désirés obtenus auparavant présentent un contenu en isomères de configuration cis inférieur à 95%.

Selon un mode d'exécution préférentiel de l'invention, l'acide (+)-(1R,2S,3R)-3-hydroxy-2-pentyl-1-cydopentaneacétique pratiquement pur obtenu à l'étape d) du procédé ci-dessus est traité avant son oxydation avec une amine de formule 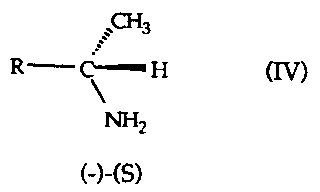 dans laquelle R représente un radical phényle ou 1-naphtyle, le sel formé étant ensuite cristallisé dans un solvant organique approprié et le sel cristallin ainsi obtenu acidifié ensuite.

Ce mode d'exécution préférentiel se révèle particulièrement avantageux lorsque l'on désire aussi éliminer toute trace d'acide (-)-(1S)-cis-3-oxo-2-pentyl-1-cyclopentaneacétique éventuellement présent dans le produit de l'hydrogénation des composés (I).

On peut ainsi obtenir l'acide (+)-(1R,2S,3R)-3-hydroxy-2-pentyl-1-cyclopentaneacétique avec une pureté d'au moins 95% et dont l'oxydation permettra d'obtenir l'acide susmentionné désiré avec une pureté similaire.

Il est clair de ce qui précède que la méthode de purification selon l'invention décrite ci-dessus est d'une application bien plus générale que la simple purification du produit d'hydrogénation susmentionné. Elle constitue en effet un procédé alternatif pour la préparation de l'acide (+)-(1R)-cis-3-oxo-2-pentyl-1-cydopentaneacétique, ou de son ester méthylique, à l'état pur, à partir de l'Hédione ® racémique ou optiquement active et sous forme de n'importe quel mélange isomérique cis/trans. Grâce aux étapes originales essentielles de ce procédé, à savoir la réduction sélective de l'Hédione ® de départ à l'aide de L-Selectride ® et la séparation des énantiomères de l'acide c-3-hydroxy-c-2-pentyl-r-1-cyclopentaneacétique à l'aide des amines susmentionnées et de leurs énantiomères, on peut ainsi préparer l'acide (+)-(1R)-cis-3-oxo-2-pentyl-1-cyclopentaneacétique désiré avec une pureté d'au moins 95%. Ce dernier peut ensuite être transformé en (+)-(1R)-cis-3-oxo-2-pentyl-1-cyclopentaneacétate de méthyle pur, ou (+)-cis-Hédione ®, l'isomère préféré de cet ingrédient parfumant, comme il est décrit plus haut, pour fournir ce composé à l'état pur.

Ainsi, selon une variante de l'invention, on fournit un procédé pour la préparation de l'acide (+)-(1R)-cis-3-oxo-2-pentyl-1-cyclopentaneacétique ou du (+)-(1R)-cis-3-oxo-2-pentyl-1-cyclopentaneacétate de méthyle, ayant une pureté d'au moins 95%, caractérisé en ce qu'on oxyde l'acide (+)-(1R,2S,3R)-3-hydroxy-2-pentyl-1-cyclopentaneacétique à l'aide de chlorochromate de pyridinium, en présence d'acétate de sodium, et, le cas échéant, on estérifie dans des conditions appropriées l'acide (+)-(1R)-cis-3-oxo-2-pentyl-1-cyclopentaneacétique ainsi formé.

L'acide (+)-(1R,2S,3R)-3-hydroxy-2-pentyl-1-cyclopentaneacétique, utilisé comme produit de départ selon cette variante du procédé de l'invention, est préparé par traitement de l'acide c-3-hydroxy-c-2-pentyl-r-1-cyclopentaneacétique avec une amine de formule (V) telle que définie plus haut, suivi de cristallisation du sel ainsi formé dans un solvant organique approprié et de l'acidification du sel cristallin ainsi obtenu.

L'acide c-3-hydroxy-c-2-pentyl-r-1-cyclopentaneacétique est lui préparé à l'état pur selon une méthode qui consiste en :
a) la réduction du 3-oxo-2-pentyl-1-cydopentaneacétate de méthyle à l'aide de tri-(sec-butyl)-borohydrure de lithium;
b) le traitement du produit de la réaction avec H₂O₂ et un excès de NaOH, suivi d'acidification, pour obtenir un mélange d'acides c-3-hydroxy-c-2-pentyl-r-1-cyclopentaneacétique et t-3-hydroxy-t-2-pentyl-r-1-cyclopentaneacétique;
c) le traitement thermique dudit mélange dans le toluène, avec séparation de l'eau par distillation azéotropique, suivi d'un traitement avec carbonate de potassium pour isoler la (1RS,8SR)-8-pentyl-2-oxabicyclo [3.2.1]octan-3-one formée; et
d) le traitement de cette lactone avec NaOH, suivi d'acidification.

Comme cité auparavant, l'estérification de l'acide (+)-(1R)-cis-3-oxo-2-pentyl-1-cyclopentaneacétique pratiquement pur obtenu selon cette variante peut être effectuée à l'aide de diazométhane pour fournir le (+)-(1R)-cis-3-oxo-2-pentyl-1-cyclopentaneacétate de méthyle, ou (+)-cis-Hédione ® de pureté équivalente.

D'autres méthodes d'estérification peuvent également être utilisées, pour autant qu'elles conservent la stéréochimie de l'acide susmentionné. Selon un mode d'exécution préférentiel, on utilise le dicarbonate de diméthyle dans le méthanol. Selon un autre mode d'exécution avantageux, on emploie du sulfate de diméthyle et carbonate de potassium dans un solvant approprié, par exemple l'acétone.

n convient de noter que l'acide (+)-(1R,2S,3R)-3-hydroxy-2-pentyl-1-cydopentaneacétique, utilisé comme produit de départ dans cette variante du procédé de l'invention, peut aussi être directement méthylé, par exemple, par réaction avec un excès de diazométhane en solution éthérée, pour fournir le (+)-(1R,2S,3R)-3-hydroxy-2-pentyl-1-cyclopentaneacétate de méthyle pratiquement pur, lequel peut ensuite être transformé en (+)-cis-Hédione ® par oxydation.

Bien entendu, si désiré, les isomères optiquement actifs de configuration trans de l'Hédione ® peuvent aussi être obtenus à l'état pur par épimérisation de leurs diastéréomères (+)-(1R)-cis et (-)-(lS)-cis préparés comme il est mentionné plus haut.

Les procédés décrits ci-dessus permettent donc d'obtenir l'acide (+)-(1R)-cis-3-oxo-2-pentyl-1-cyclopentaneacétique et le (+)-(1R)-cis-3-oxo-2-pentyl-1-cyclopentaneacétate de méthyle, l'isomère préféré de l'Hédione ®, avec une pureté optique d'au moins 95% et une configuration strictement cis. Ceci grâce à des transformations pouvant être utilisées à l'échelle industrielle. Ces produits, d'une telle pureté stéréochimique et optique, sont des composés nouveaux qui font également l'objet de l'invention.

Ces procédés fournissent en plus des produits intermédiaires optiquement purs qui peuvent être obtenus à l'échelle industrielle et qui sont des composés nouveaux. L'invention a aussi pour objet ces produits intermédiaires dont l'utilisation selon l'invention permet d'obtenir les produits finaux optiquement purs cités plus haut.

L'invention sera maintenant décrite plus en détail à l'aide des exemples suivants, dans lesquels les températures sont indiquées en degrés centigrades et les abréviations ont le sens usuel dans l'art.

### Manières de réaliser l'invention

### Exemple 1

### Préparation de l'acide (+)-(1R)-cis-3-oxo-2-pentyl-1-cyclopentaneacétique

a) (1RS,8SR)-8-pentyl-2-oxabicyclo[3.2.1]octan-3-one
   Dans un ballon de 2l à 3 cols et double paroi, équipé d'un thermostat, d'un agitateur mécanique et d'une ampoule d'introduction, maintenu sous argon, on a introduit une solution dans le tétrahydrofurane (THF; 1 M; 200 ml) de L-Sélectride ® via canule dans le ballon. On a agité, maintenu à -20° et introduit goutte à goutte une solution d'Hédione ® (3-oxo-2-pentyl-1-cyclopentaneacétate de méthyle; 40 g; 177 mmole ; mélange cis/trans 67:33) dans 82 ml de THF pendant ca. 35 min. On a agité 2 h à -20° et monté à 0°. On a introduit goutte à goutte du NaOH aq. (3N; 250 ml). Après avoir interrompu le courant de Ar, on a introduit goutte à goutte du H₂O₂ (30%; 250 ml; Fluka). Après avoir chauffé et agité une nuit à 65°, on a laissé refroidir et enlevé la plupart du THF au rotavapeur.
   La solution aq. alcaline résultante a été extraite à l'éther 2x. On a acidifié la phase aq. alcaline avec HCl 3N, extrait 2x à l'éther, séché sur MgSO₄ et concentré. On a repris dans 100 ml de toluène et chauffé à reflux avec un séparateur d'eau pendant 18 h pour cycliser en lactone. Après concentration, on a obtenu 29,1 g d'un mélange brut contenant la lactone désirée.
   On a repris ce mélange dans l'éther et extrait 2x avec 100 ml de K₂CO₃ 1M aq. Les phases aqueuses ont été extraites 3x à l'éther et les phases éthérées ont été lavées à l'eau jusqu'à neutralité. On a séché sur MgSO₄ et concentré. On a obtenu 14,1 g de lactone brute. Après distillation au four à boules sous 13 Pa à 170°, on a obtenu 11,3 g de (1RS,8SR)-8-pentyl-2-oxabicyclo[3.2.1]octan-3-one (pureté : 97%; 58 mmole; rend. 48%).
   RMN(¹H,360MHz): 0,89(t,J=7,3H); 1,20-1,63(8H); 1,63-1,78(2H); 1,87-2,03(2H); 2,06-2,18(1H); 2,36(d,J=20, 1H, recouvert par s large, 2,32, 1H); 2,74(dxdxd, J=20, 2,0, 1,6, 1H); 4,61(s large, 1H) δ ppm
   RMN(¹³C): 14,0(q); 22,6(t); 25,4(t); 28,0(t); 30,0(t); 31,9(t); 32,2(t); 34,5(d); 36,2(d); 44,8(d); 82,4(d); 171,1(s) δ ppm
   SM : 196(2,M⁺), 98(50), 84(95), 83(100), 82(81), 81(75), 67(50), 55(59)
   Odeur : lactonique, champignon, fruitée, fleurie

   Toutes les phases aqueuses de l'opération ci-dessus ont été réunies et acidifiées avec HCl 1N, extraites 3 fois à l'éther, séchées sur MgSO₄ et concentrées au rotavapeur pour fournir 12,4 g de l'acide t-3-hydroxy-t-2-pentyl-r-1-cyclopentaneacétique brut.
b) acide c-3-hydroxy-c-2-pentyl-r-1-cyclopentaneacétique
   Dans un ballon, on a chargé 16,08 g (82,0 mmole) de la lactone obtenue sous a), 33 ml d'une solution aq. de NaOH (99 mmole) et 57 ml de THF. On a agité et chauffé à 70° pendant 4,5 h. La plupart du THF a été enlevé par distillation. On a refroidi et extrait 2 fois à l'éther, en éliminant ces extraits. La phase aqueuse a été acidifiée avec HCl aq. 3N. On a extrait 2 fois à l'éther, lavé les extraits combinés à l'eau, séché sur MgSO₄, filtré et concentré. Le résidu brut ainsi obtenu (16,2 g; rend. 82%) a formé des cristaux (p. f. 74-76°). L'acide désiré ainsi obtenu peut être utilisé tel quel dans l'étape suivante ou recristallisé dans le cydohexane/acétate d'éthyle pour fournir un produit ayant p. f. 79-81° (cristaux incolores).
   RMN(¹H,360MHz): 0,89(t, J=6, 3H); 1,22-1,53(8H); 1,57-1,95(5H); 2,32-2,53 (3H); 4,22(t, J=3,5, 1H); ca 5,9-7,0(large, 2 OH) δ ppm
   RMN(¹³C): 14,1(q); 22,6(t); 25,1(t); 28,3(t); 29,5(t); 32,3(t); 33,6(t); 36,3(d); 36,5(t); 47,9(d); 74,8(d); 179,9(s) δ ppm
   SM : 84(82), 83(98), 82(70), 81(71), 79(56), 77(63), 55(94), 41(100)
c) acide (+)-(1R,2S,3R)-3-hydroxy-2-pentyl-1-cyclopentaneacétique et acide (-)-(1S,2R,3S)-3-hydroxy-2-pentyl-1-cyclopentaneacétique
   L'acide cristallin obtenu sous b) (8,63 g; 40,3 mmole) a été dissous dans l'éthanol (190 ml) et on a ajouté de la (+)-(R)-1-phényléthylamine [4,88 g; 40,3 mmole; Fluka purum, ratio énantiomérique (r.e.) 98 : 2]. On a concentré au rotavapeur et dissous le résidu dans 300 ml d'un mélange chaud de cyclohexane/acétate d'éthyle 5 : 1 (v/v), puis on a laissé cristalliser pendant la nuit. Après filtration et séchage à l'air, on a obtenu 5,93 g de produit cristallin ayant p. f. 127-130°. Ce produit a été recristallisé dans 250 ml d'un mélange de solvants identiques à celui susmentionné pour fournir 4,78 g de (1S,2R,3S)-3-hydroxy-2-pentyl-1-cyclopentaneacétate de (R)-1-phényléthylammonium [p. f. 132-135°; [α]²⁰_{D} = -4° (c=1,10 g /100 ml, éthanol)]. Ce sel a été repris à l'éther et extrait une fois avec 35 ml de NaOH 1N. La phase éthérée a été lavée 2x à l'eau et les phases aqueuses combinées extraites à l'éther. Le séchage des phases organiques combinées sur MgSO₄, la filtration et la concentration fournissent la (+)-(R)-1-phényléthylamine qui peut être recyclée.
   Les phases aqueuses combinées ont été acidifiées avec 40 ml de HCl aq. (1N) et extraites 2x à l'éther. On a séché sur MgSO₄, filtré et concentré pour obtenir 2,80 g d'acide (-)-(1S,2R,3S)-3-hydroxy-2-pentyl-1-cyclopentaneacétique, sous forme d'une huile incolore (13,1 mmole; rend. 92%).
   Cet acide présentait les caractères analytiques de l'acide racémique décrit sous b) et un [α]²⁰_{D} = -12° (c=1,15, éthanol) (pureté: 99%; r.e. 99,2:0,8).
   En procédant de façon analogue à celle décrite ci-dessus mais en utilisant la (-)-(S)-1-phényléthylamine (Fluka purum; r.e. 98 : 2), on a obtenu le (1R,2S,3R)-3-hydroxy-2-pentyl-1-cyclopentaneacétate de (S)-1-phényléthylammonium (p. f. 131-132°; [α]²⁰_{D} = +4°; c=1,05, éthanol) et l'acide (+)-(1R,2S,3R)-3-hydroxy-2-pentyl-1-cyclopentaneacétique ayant un [α]²⁰_{D} = +11° (c=1,10, éthanol) (pureté: 99% ; r.e. 96,5:3,5).
   Ces expériences ont été répétées avec (+)-(R)-1-(1-naphtyl)éthylamine (Fluka purum; r.e. > 99 : 1), qui a fourni le (1S,2R,3S)-3-hydroxy-2-pentyl-1-cyclopentaneacétate de (R)-1-(1-naphtyl)éthylammonium (p. f. 134-136° ; [α]²⁰_{D} = +4° (c=1,145, éthanol), menant à l'acide (-)-(1S,2R,3S)-3-hydroxy-2-pentyl-1-cyclopentaneacétique. La configuration absolue de cet acide a été confirmée par rayons X du sel intermédiaire.
   D'autre part, la (-)-(S)-1-(1-naphtyl)éthylamine (Fluka purum; r.e. > 99 : 1) a fourni le (1R,2S,3R)-3-hydroxy-2-pentyl-1-cyclopentaneacétate de (S)-1-(1-naphtyl)éthylammonium [p. f. 134-135°; [α]²⁰_{D} = -4° (c=1,05, éthanol)] et l'acide (+)-(1R,2S,3R)-3-hydroxy-2-pentyl-1-cyclopentaneacétique.
d) acide (+)-(1R)-cis-3-oxo-2-pentyl-1-cyclopentaneacétique et acide (-)-(1S)-cis-3-oxo-2-pentyl-1-cyclopentaneacétique
   Ces acides ont été préparés à partir des hydroxyacides décrits sous c), comme suit.
   On a chargé un ballon équipé d'un thermomètre et d'un manchon de refroidissement avec 5,66 g de PCC (26,3 mmole), 322 mg d'acétate de sodium anhydre (3,93 mmole) et 30 ml de CH₂Cl₂. On a refroidi la suspension à -10°, agité et ajouté l'hydroxyacide (∼ 13 mmole) dans 10 ml de CH₂Cl₂, tout à la fois. On a agité à -10° pendant 20 h. On a passé le mélange réactionel à travers une colonne de SiO₂ dans l'éther, ainsi que la solution de CH₂Cl₂ et les solutions de lavage du ballon de réaction (à l'éther). On a concentré les solutions éluées pour obtenir l'acide (+)-(1R)-cis-3-oxo-2-pentyl-1-cyclopentane-acétique avec une pureté de 95% et un rapport énantiomérique de 99:1.
   Les caractères analytiques de ce composé étaient les suivants:
   [α]²⁰_{D} = +71° (c=0,985, éthanol)
   RMN(¹H,360MHz): 0,88(t, J=7, 3H) ; 1,17-1,47(7H); 1,55-1,69(1H); 1,80-1,95(1H); 1,98-2,22(1H); 2,13(dxd, J=15, 10, 1H); 2,20-2,37(3H); 2,46 (dxd,J=15, 5, 1H); 2,84(septet large, J=5, 1H) δ ppm
   RMN(¹³C): 14,0(q); 22,5(t); 24,6(t); 25,6(t); 27,1(t); 31,8(t); 33,7(t); 35,1(t); 35,5(d); 52,6(d); 178,6(s); 219,5(s) δ ppm
   SM : 212(2,M⁺), 153(20), 142(31), 84(7), 83(100), 82(21), 67(6), 55(8)

   Son énantiomère de configuration (-)-(1S) a aussi été obtenu.

### Exemple 2

### Préparation de l'acide (+)-(1R)-cis-3-oxo-2-pentyl-1-cyclopentaneacétique

Dans un ballon de type Schlenk, sec, sous Ar, on a injecté la solution précatalytique (6,75 ml) contenant 47 mg (0,05 mmole) de bis(trifluoroacétato)[(+)-(R)-2,2'-bis-(diphénylphosphino)-1,1'-binaphtyl]ruthénium (II), l'acide 3-oxo-2-pentyl-1-cyclopentène-1-acétique (0,5 g, 2,4 mmole), du CH₂Cl₂ (5 ml), du méthanol (5 ml) et de la triéthylamine (10,28 mg, 0,1 mmole, 14 µl, dist. sur KOH). On a agité 5 min et filtré la solution orange à travers un filtre à usage unique (Acrodisc 0,45 µm) dans un autoclave (100 ml/10⁷ Pa) sec et sous Ar. L'autoclave a ensuite été branché sur une source d'hydrogène et rincé 5x à l'hydrogène sous une pression de 10⁶ Pa. Ensuite, l'autoclave a été pressurisé à 9x10⁶ Pa. On a agité à cette pression et 25° pendant 120 h. Après, on a relaché gentiment la pression, ouvert l'autoclave et concentré la solution orange.

L'analyse de l'acide (+)-(1R)-cis-3-oxo-2-pentyl-1-cydopentaneacétique ainsi obtenu a été effectuée via le (+)-(1R)-cis-3-oxo-2-pentyl-1-cyclopentaneacétate de méthyle obtenu par addition de diazométhane en solution éthérée. Après le traitement usuel, l'analyse par chromatographie gaz-liquide sur colonne chirale (cyclodextrine alkylée d'origine commerciale) de l'ester ainsi obtenu a montré qu'il s'agissait d'un mélange ayant 85% en poids d'isomères de configuration cis, dont 85% de (+)-(1R) et 15% de (-)-(1S) (excès énantiomérique e.e. 70%), et 15% d'impuretés, dont environ 5% d'isomères de configuration trans dudit acide.

Des expériences identiques mais en utilisant la triéthylamine dans des rapports molaires compris entre 1,4:1 et 4 : 1, par rapport au catalyseur ont fourni le produit final avec une pureté optique équivalente.

Le catalyseur utilisé a été préparé au préalable in situ, selon la méthode décrite par B. Heiser, réf. citée, ainsi.
Ru(COD)(η³-méthallyl)₂ (origine: Intex Chemikalien, Muttenz, Suisse; 16 mg, 0,05 mmole) a été mélangé sous Ar et dans des conditions anhydres avec de l'acide trifluoroacétique (11,4 mg, 0,1 mmole) et du méthanol (0,25 ml). A la suspension résultante, on a ajouté une solution de (+)-(R)-2,2'-bis(diphénylphosphino)-1,1'-binaphtalène (R-BINAP ; origine : Fluka ; 31 mg, 0,05 mmole) dans un mélange de méthanol (3 ml) et dichlorométhane (3,5 ml) et laissé réagir sous agitation à température ambiante pendant 3 jours. La solution jaune claire contenant le catalyseur (7,4 mmole/l) a été utilisée telle quelle dans l'hydrogénation.

L'acide 3-oxo-2-pentyl-1-cyclopentène-1-acétique utilisé comme produit de départ dans cette hydrogénation a été préparé comme suit, par saponification du 3-oxo-2-pentyl-1-cyclopentène-1-acétate de méthyle [obtenu comme il est décrit dans US 5,302,745 ou EP-A1-583 564].
On a dissous du LiOH (9,48 g; 0,4 mole) dans de l'eau déminéralisée (160 ml). On a rajouté une solution de 3-oxo-2-pentyl-1-cyclopentène-1-acétate de méthyle (10 g; 94% ; 42 mmole) dans du THF (200 ml) et 10 gouttes d'éthanol. Le mélange résultant a été agité pendant 24 h à température ambiante. Après, on a versé sur 1000 ml d'eau déminéralisée refroidie à 0-5° et neutralisé avec du HCl concentré. Par extraction au dichlorométhane, on a obtenu 9 g d'une huile brute contenant environ 85% d'acide 3-oxo-2-pentyl-1-cyclopentène-1-acétique.
RMN(¹H,360MHz): 0,87(t,3H); 1,2-1,4(m, 6H); 2,2(t, 2H); 2,45(m, 2H); 2,66(m, 2H); 3,5(s, 2H); 8,4(s large, 1H) δ ppm
RMN(¹³C): 13,95(q); 22,44(t); 23,23(t); 27,97(t); 29,84(t); 31,79(t); 34,37(t); 36,56(t); 143,54(s); 163,86(s); 173,65(s); 210,01(s) δ ppm

### Exemple 3

### Préparation de l'acide (-)-(1S)-cis-3-oxo-2-pentyl-1-cyclopentaneacétique

Préparé de façon identique à celle décrite dans l'Exemple 2, par hydrogénation de l'acide 3-oxo-2-pentyl-1-cyclopentène-1-acétique, en présence de bis(trifluoroacétato)[(-)-(S)-2,2'-bis-(diphénylphosphino)-1,1'-binaphtyl] ruthénium (II) [ce dernier a été obtenu comme il est décrit dans l'Exemple 2, à partir de Ru(COD)(η³-méthallyl)₂ et de S-BINAP (origine: Fluka)]. L'acide (-)-(1S)-cis-3-oxo-2-pentyl-1-cyclopentaneacétique présentait une pureté analogue à celle de son énantiomère décrit dans l'Exemple 2.

### Exemple 4

### Préparation de (+)-(1R)-cis-3-oxo-2-pentyl-1-cyclopentaneacétate de méthyle

A. Par méthylation de l'acide (+)-(1R,2S,3R)-3-hydroxy-2-pentyl-1-cyclopentaneacétique décrit dans l'Exemple 1 c) et oxydation subséquente du produit obtenu
   On a ajouté lentement à l'acide susmentionné (2,67 g; 12,5 mmole) dissous dans 5 ml d'éther, refroidi à l'aide d'un bain de glace, une solution de diazométhane dans l'éther (0,2 M), jusqu'à persistance de la couleur jaune. Après addition d'un petit excès encore de cette solution, on a laissé pendant la nuit à température ambiante. Après évaporation du solvant, on a obtenu 2,85 g de (+)-(1R,2S,3R)-3-hydroxy-2-pentyl-1-cyclopentaneacétate de méthyle (pureté ∼ 99%; r.e. 96,5:3,5).
   [α]²⁰_{D} = +17° (c=1,795, méthanol)
   RMN(¹H,360MHz): 0,89(t, J=7, 3H) ; 1,22-1,50(8H); 1,53-1,93(5H); 2,32-2,52(3H); 3,67(s, 3H); 4,20(dxt, J=4, 1,5, 1H) δ ppm
   RMN(¹³C): 14,1(q); 22,7(t); 25,1(t); 28,4(t); 29,5(t); 32,3(t); 33,7(t); 36,4(d); 36,6(t); 47,9(d); 51,4(q); 74,8(d); 174,8(s) δ ppm
   SM : 136(62), 84(63), 83(79), 81(71), 74(100), 67(58), 55(66), 41(62)

   Cet ester (2,85 g) a ensuite été oxydé à l'aide de PCC, en présence d'acétate de sodium, de façon analogue à la méthode décrite dans l'Exemple 1 d), pour fournir 2,68 g du (+)-(1R)-cis-3-oxo-2-pentyl-1-cyclopentaneacétate de méthyle désiré (huile incolore; pureté 98,5%; rend. 92,7%).
   [α]²⁰_{D} = +69° (c=1,49, méthanol); +83,5° (c=1,19, chloroforme)
   rapport cis/trans 95:5
   r.e. 97,0:3,0
   RMN(¹H,360MHz): 0,89(t, J=6,7, 3H); 1,13-1,45(7H); 1,52-1,69(1H); 1,77-1,88(m, 8 lignes, 1H); 1,95-2,08(1H) ; 2,08(dxd, J=15,4, 10,1, 1H); 2,14-2,33(3H); 2,39(dxd, J=15,4, 5,3, 1H); 2,83(septet large, J=5,3), 3,70(s, 3H) δ ppm
   RMN(¹³C): 14,0(q); 22,5(t); 24,7(t); 25,7(t); 27,2(t); 31,9(t); 33,7(t); 35,1(t); 35,8(d); 51,7(q); 52,7(d); 173,0(s); 219,2(s) δ ppm
   SM : 226(8,M⁺), 156(29), 153(29), %(11), 83(100), 82(31), 55(23), 41(22)
B. Par méthylation de l'acide (+)-(1R)-cis-3-oxo-2-pentyl-1-cyclopentane-acétique décrit dans l'Exemple 1
   En appliquant à cet acide le traitement avec diazométhane analogue à celui décrit sous A., on a obtenu le produit désiré avec des caractéristiques semblables à celles décrites ci-dessus.
C. Par méthylation de l'acide (+)-(1R)-cis-3-oxo-2-pentyl-1-cyclopentaneacétique décrit dans l'Exemple 2
   On a ajouté à l'acide (+)-(1R)-cis-3-oxo-2-pentyl-1-cyclopentaneacétique obtenu selon l'Exemple 2 (résidu résultant de la concentration de la solution orange) un excès de diazométhane sous forme d'une solution à 2% dans l'éther sulfurique et agité pendant 15 min. Après concentration au rotavapeur, on a ajouté du pentane (12 ml) pour précipiter le catalyseur. On a filtré sur 150 mg de silica gel (0,04-0,063 mm) dans une pipette pasteur et rincé avec du pentane (12 ml). Le filtrat a été concentré. Ainsi, on a obtenu 0,5 g d'un mélange contenant 85% de (+)-(1R)-cis-3-oxo-2-pentyl-1-cyclopentaneacétate de méthyle avec un excès énantiomérique de 70% (déterminé par une analyse chromatographique gaz-liquide sur colonne chirale).
   Le produit ainsi obtenu a pu être purifié en suivant le procédé décrit dans l'Exemple 1, pour obtenir un acide (+)-(1R)-cis-3-oxo-2-pentyl-1-cyclopentaneacétique de départ plus pur, lequel a ensuite été méthylé à l'aide de diazométhane, de façon analogue à celle décrite ci-dessus, pour fournir le (+)-(1R)-cis-3-oxo-2-pentyl-1-cyclopentaneacétate de méthyle avec une pureté identique à celle du produit décrit sous A.

### Exemple 5

### Préparation de (-)-(1S)-cis-3-oxo-2-pentyl-1-cyclopentaneacétate de méthyle

Ce composé a été préparé à partir de l'acide (-)-(1S,2R,3S)-3-hydroxy-2-pentyl-1-cydopentaneacétique [voir Exemple 1 c)] de façon analogue à celle décrite dans l'Exemple 4 A.
Le (-)-(1S)-cis-3-oxo-2-pentyl-1-cydopentaneacétate de méthyle (huile incolore; pureté 98,6%) possédait des caractères analytiques identiques à ceux de son énantiomère de configuration (+)-(1R)-cis, à l'exception de :
[α]²⁰_{D} = - 66° (c=1,39, méthanol) ; - 88° (c=1,24, chloroforme)
rapport cis/trans 94 : 6
r.e. 98,0:2,0

Le (-)-(1S,2R,3S)-3-hydroxy-2-pentyl-1-cyclopentaneacétate de méthyle intermédiaire possédait un [α]²⁰_{D} = -16° (c=1,805, méthanol)

### Exemple 6

### Préparation de (+)-(1S)-trans-3-oxo-2-pentyl-1-cyclopentaneacétate de méthyle et de (-)-(1R)-trans-3-oxo-2-pentyl-1-cyclopentaneacétate de méthyle

Ces deux isomères optiquement actifs de l'Hédione® ont été préparés par épimérisation de leurs diastéréomères respectifs décrits dans les Exemples 4 et 5, à l'aide de KNH₂ dans NH₃ liquide à -75°.

Les composés obtenus présentaient des caractères analytiques identiques à ceux décrits dans la littérature [T. Kitahara et al., Agric. Biol. Chem. 50, 1867 (1986)], excepté :
isomère de configuration (-)-(1R)-trans
   [α]²⁰_{D} = - 41° (c=1,15, méthanol); -50° (c=1,20, chloroforme)
   pureté : 99,9%
   rapport cis/trans 4 : 96
   r. e. 93,2 : 6,8
isomère de configuration (+)-(1S)-trans
   [α]²⁰_{D} = +46° (c=1,08, méthanol); +55° (c=1,36, chloroforme)
   rapport cis/trans 4 : 96
   r. e. 97,4 : 2,6

### Exemple 7

### Préparation de l'acide (+)-(1R)-3-oxo-2-pentyl-1-cyclopentaneacétique et du (+)-(1R)-3-oxo-2-pentyl-1-cyclopentaneacétate de méthyle

a) Préparation du 3-oxo-2-pentyl-1-cyclopentène-1-acétate de lithium
   On a mélangé dans l'eau (1 ml) l'acide 3-oxo-2-pentyl-1-cyclopentène-1-acétique (0,5 g; 2,38 mmole) et du Li₂CO₃ (Fluka; 0,0876 g; 1,18 mmole). Après 1 h d'agitation, on a obtenu une solution limpide qui a été concentrée à sec. Les cristaux résiduels ont ensuite été dissous dans 2 ml de méthanol et la solution obtenue ajoutée à 20 ml d'éther isopropylique pour cristallisation. Les cristaux ont été filtrés et séchés sous vide pendant 60 min. On a ainsi obtenu l'acétate de lithium susmentionné sous forme de cristaux blancs très stables (décomposition 190°) dont les données analytiques étaient les suivantes:
   RMN(¹H,360MHz,H₂0): 0,87(t, 3H); 1,1-1,4(m, 6H); 2,17(t, 2H); 2,47(m, 2H) ; 2,68(m, 2H) ; 3,45(s, 2H) δ ppm
   RMN(¹³C,H₂0): 16,14(q); 24,65(t); 25,07(t); 30,11(t); 33,05(t); 33,7(t); 37,19(t); 43,23(t); 143,89(s); 176,%(s); 179,76(s); 218,28(s) δ ppm
   SM:193(2), 167(100), 151(54), 137(13), 123(11), 110(57), 95(10), 91(4)

   La structure du produit a aussi été confirmée par spectroscopie de masse de type "electron spray".
   Les autres acétates analogues ont été préparés de façon identique, à l'aide des carbonates appropriés, c'est-à-dire de sodium, de potassium, de césium, d'ammonium, de triéthylammonium et autres.
   Le composé décrit plus haut sera généralement préparé in situ par mélange de l'acide de départ et Li₂CO₃ dans l'eau, comme décrit plus haut, la solution limpide obtenue après 1 h d'agitation étant utilisée telle quelle dans l'hydrogénation.
b) Préparation du bis(trifluoroacéto)[(+)-(R)-BINAP-sulfoné]ruthénium (II)
   On a préparé le (+)-(R)-BINAP-sulfoné à partir du (+)-(R)-BINAP ou (+)-(R)-2,2'-bis(diphénylphosphino)-1,1-binaphtalène (voir Exemple 2), en procédant de façon identique à celle décrite par Kam-to Wan et al. (réf. citée) et illustrée au schéma suivant: 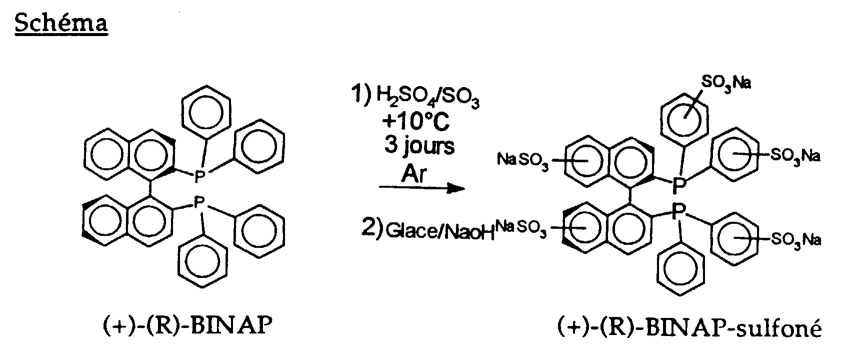
   La structure du (+)-(R)-BINAP-sulfoné est représentée en accord avec l'hypothèse formulée par les auteurs cités. On peut, par ailleurs, en modifiant les conditions de la réaction, augmenter la sulfonation du BINAP, c'est-à-dire le nombre de groupes SO₃Na qui lui sont liés.
   Le (+)-(R)-BINAP-sulfoné obtenu (112 mg) a été ajouté à 4 ml de méthanol et 2 ml d'eau et la solution agitée pendant 24 h à température ambiante, sous argon. Cette solution a ensuite été mélangée à la suspension obtenue comme il est décrit à l'Exemple 2 par réaction de Ru(COD)(η³-méthallyl)₂ et acide trifluoroacétique. On a agité le mélange pendant 3 jours à température ambiante et sous Ar pour obtenir une solution orange-brune presque limpide contenant le catalyseur désiré et qui a été utilisée telle quelle dans l'hydrogénation.
   Le (-)-(S)-BINAP-sulfoné et le complexe de Ru(II) correspondant ont été préparés de façon analogue.
c) Les solutions obtenues sous a) et b) ont été mélangées et le mélange agité pendant 3-5 jours sour Ar. La solution résultante a été chargée dans un autoclave et hydrogénée à 90x10⁵ Pa et t. a. pendant 42h jusqu'à une conversion de 97%. On a décomprimé l'autoclave, prélevé le mélange réactionnel et ramené le pH à 3,5 en rajoutant de l'HCl 1N. Ce mélange a été extrait avec l'éther sulfurique une fois avec (5 ml) et une fois avec (3 ml). L'extrait éthéré a été traité avec un excès de diazométhane et ensuite concentré. Du pentane (1 ml) et une spatule de Na₂SO₄ ont été rajoutés au résidu et on a agité 5 min. On a filtré et concentré. On a ainsi obtenu 0,5 g de (+)-(IR)-3-oxo-2-pentyl-1-cydopentaneacétate de méthyle pur (la chromatographie de ce produit indiquait un rapport cis/trans 95:5; rapport (+)-(1R)-cis/(-)-(1S)-cis 95:5, donc 90% e.e.).
   L'acide (+)-(1R)-cis-3-oxo-2-pentyl-1-cyclopentaneacétique obtenu sous forme de la solution éthérée susmentionnée (avant le traitement avec diazométhane) avait les mêmes caractéristiques de pureté que son ester méthylique susmentionné.
   De façon analogue, on a préparé les énantiomères de ces produits avec une pureté comparable.

### Exemple 8

### Préparation de l'acide (+)-(1R)-cis-3-oxo-2-pentyl-1-cyclopentaneacétique et de (+)-(1R)-cis 3-oxo-2-pentyl-1-cyclopentaneacétate de méthyle

a) Préparation du bis(trifluoroacéto)[(-)-(R,R)-Et-DuPHOS]ruthénium (II)
   Une solution de Ru(COD)(η³-méthallyl)₂ (48 mg ; 0,15 mmole) dans 3 ml de tétrahydrofuran (THF) a été traitée avec 46 µl (0,60 mmole, 4 éq.) de CF₃COOH et la solution résultante a été agitée pendant 2 h à t. a. On a enlevé le THF et l'excès de CF₃COOH sous vide poussé et repris le solide jaune ainsi obtenu dans 3 ml de THF. On a ajouté 54,3 mg (0,15 mmole) de (-)-Et-DuPHOS (origine: Strem) et la solution résultante a été laissée sous agitation à t. a. pendant 4 jours, protégée de la lumière. On a ensuite ajouté 9 ml de méthanol et la solution obtenue contenant le catalyseur désiré a été utilisée telle quelle dans l'hydrogénation suivante. Toutes les manipulations ont eu lieu sous Ar et avec solvants dégasés.
b) On a ajouté à une solution de 252 mg (1,20 mmole) d'acide 3-oxo-2-pentyl-1 cyclopentène-1-acétique, dans un mélange de 7 ml de THF et 21 ml de méthanol, 2 ml de la solution du catalyseur obtenue en a) (0,025 mmole, 0,021 eq.) et 10,4 µl de triéthylamine (0,075 mmole, 3 eq.) sous agitation à t. a. Cette solution, préparée dans le récipient en verre d'un autoclave standard, a été introduite dans cet autoclave, lequel a été scellé, mise sous une pression de 45x10⁵ Pa d'hydrogène et agitée à t. a. La réaction a été suivie par chromatographie en phase gazeuse et était complète après environ 4 h. Après 1 nuit, on a évaporé le contenu, sous vide à t. a., repris le résidu à l'éther, lavé avec HCl 0,1N et ensuite eau, séché sur MgSO₄ et filtré. L'acide (+)-(1R)-3-oxo-2-pentyl-1-cyclopentaneacétique ainsi obtenu a été estérifié à l'aide de diazométhane pour fournir le (+)-(1R)-cis-3-oxo-2-pentyl-1-cyclopentaneacétate de méthyle dont l'analyse chromatographique (GC) indiquait une pureté de 97%, rapport cis/trans 96:4 et rapport (+)-(1R)-cis/(-)-(1S)-cis 95:5.

### Exemple 9

### Préparation de (±)-cis-3-oxo-2-pentyl-1-cyclopentaneacétate de méthyle

a) On a traité 104 mg (0,49mmole) d'acide (±)-cis-3-oxo-2-pentyl-1-cyclopentaneacétique (rapport cis/trans 97:3) dans 0,5 ml de méthanol avec 76 µl de dicarbonate de diméthyle [CH₃O(CO)O(CO)OCH₃, origine : Bayer ; 96 mg, 0,72 mmole, 1,5 éq.]. La solution résultante a été chauffée à 45° pendant 16 h et ensuite évaporée sous vide. Après distillation du résidu dans un four à boules (t. four 150°/10 Pa), on a obtenu 100 mg (0,44 mmole, rend. 90%) de (±)cis-3-oxo-2-pentyl-1-cyclopentaneacétate de méthyle pur (rapport cis/trans 97:3).
b) On a ajouté à 104 mg (0,49 mmole) d'acide (±)cis-3-oxo-2-pentyl-1-cyclopentaneacétique (rapport cis/trans 93:7) dans 1 ml de (CH₃)₂CO d'abord 56 µl de (CH₃)₂SO₄ (74 ml, 0,59 mmole, 1,2 éq.) et ensuite 81 mg (0,59 mmole, 1,2 éq.) de K₂CO₃ solide anhydre. La suspension résultante a été chauffée à t. a. pendant 4 h et reprise dans l'eau et à l'éther. La phase éthérée a été séparée et la phase aqueuse extraite à nouveau à l'éther. Les phases organiques combinées ont été lavées à l'eau, séchées sur MgSO₄, filtrées et concentrées. Le résidu a été distillé au four à boules (t. four: 180°/7 Pa) pour fournir 93 mg (0,44 mmole, rend. 84%) de (±)-cis-3-oxo-2-pentyl-1-cyclopentaneacétate de méthyle (rapport cis/trans 92/8).
   Lorsqu'on a pris comme produit de départ dans les procédés décrits ci-dessus un acide optiquement actif tel qu'obtenu dans l'Exemple 8, on a obtenu le (+)-(1R)-cis-3-oxo-2-pentyl-1-cyclopentaneacétate de méthyle avec les mêmes caractéristiques stéréochimiques et optiques que le même produit décrit dans l'Exemple 8.

## Revendications

1. Procédé pour la préparation de l'acide 3-oxo-2-pentyl-1-cydopentaneacétique ou du 3-oxo-2-pentyl-1-cyclopentaneacétate de méthyle, essentiellement sous forme de leur isomère de configuration (+)-(1R)-cis, caractérisé en ce qu'il comprend les étapes suivantes:
a) l'hydrogénation catalytique, dans un solvant approprié et en présence d'un catalyseur constitué par un complexe de Ru (II) comportant des ligands chiraux qui contiennent un radical de type 2,2'-bis(diphénylphosphino)-1,1'-binaphtyle (BINAP) ou de type 1,2-bis(2,5-dialkylphospholano) benzényle (DuPHOS), l'alkyle étant un radical de C₂ ou C₃, d'un composé de formule 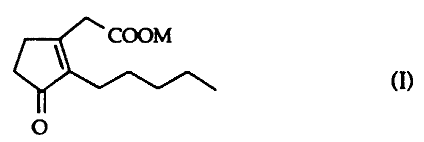 dans laquelle le symbole M représente un atome d'hydrogène, un atome d'un métal alcalin ou alcalino-terreux ou un groupe NR₄, R représentant l'hydrogène ou un radical alkyle inférieur, pour obtenir un composé de formule 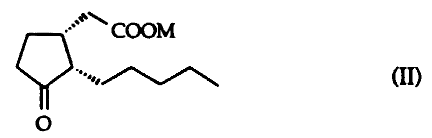 dans laquelle M a le sens indiqué ci-dessus, essentiellement sous forme de l'isomère de configuration (+)-(1R)-cis;
b) le cas échéant, l'acidification, de façon connue en soi, du composé de formule (II) dans laquelle M représente un atome d'un métal alcalin ou alcalino-terreux ou un groupe NR₄, R représentant l'hydrogène ou un radical alkyle inférieur, pour obtenir l'acide (+)-(1R)-cis-3-oxo-2-pentyl-1-cyclopentaneacétique ; et
c) le cas échéant, l'estérification, dans des conditions appropriées, de l'acide (+)-(1R)-cis-3-oxo-2-pentyl-1-cyclopentaneacétique ainsi obtenu pour former le (+)-(1R)-cis-3-oxo-2-pentyl-1-cydopentaneacétate de méthyle.

2. Procédé selon la revendication 1, caractérisé en ce qu'on soumet l'acide 3-oxo-2-pentyl-1-cyclopentène-1-acétique à hydrogénation catalytique dans un solvant organique, en présence du bis(trifluoroacétato)[(+)-(R)-BINAP]ruthénium (II) ou du bis(trifluoroacétato)[(-)-(R,R)-Et-DuPHOS] ruthénium (II).

3. Procédé selon la revendication 2, caractérisé en ce que l'hydrogénation catalytique a lieu en présence d'une base organique azotée.

4. Procédé selon la revendication 3, caractérisé en ce la base organique est la triéthylamine.

5. Procédé selon la revendication 4, caractérisé en ce que la triéthylamine est utilisée dans un rapport molaire de 2:1 à 4:1, par rapport au catalyseur de Ru (II).

6. Procédé selon l'une des revendications 2 à 5, caractérisé en ce que le solvant organique est un mélange de dichlorométhane ou tétrahydrofurane et méthanol.

7. Procédé selon la revendication 1, caractérisé en ce qu'on soumet à hydrogénation catalytique, dans un solvant approprié et en présence d'un catalyseur constitué par un complexe de ruthénium (II) et (+)-(R)-BINAP-sulfoné, un composé de formule (I) dans laquelle M représente un atome d'un métal alcalin ou alcalino-terreux ou un groupe NR₄, R représentant l'hydrogène ou un radical alkyle inférieur.

8. Procédé selon la revendication 7, caractérisé en ce que le composé de formule (I) est le 3-oxo-2-pentyl-1-cyclopentène-1-acétate de lithium et le catalyseur est le bis(trifluoroacétato)[(+)-(R)-BINAP-sulfoné]ruthénium (II).

9. Procédé selon la revendication 7 ou 8, caractérisé en ce que le solvant est l'eau ou un mélange d'eau et méthanol.

10. Procédé selon l'une des revendications précédentes, caractérisé en ce qu'il comporte en plus la purification de l'acide (+)-(1R)-cis-3-oxo-2-pentyl-1-cyclopentaneacétique ou du (+)-(1R)-cis-3-oxo-2-pentyl-1-cyclopentaneacétate de méthyle obtenus, laquelle purification comprend:
a) la réduction à l'aide de tri-(sec-butyl)-borohydrure de lithium de (+)-(1R)-cis-3-oxo-2-pentyl-1-cyclopentane-acétate de méthyle ;
b) le traitement du mélange réactionnel ainsi obtenu avec H₂O₂ et un excès de NaOH et l'acidification subséquente du sel résultant pour obtenir un mélange contenant essentiellement l'acide (+)-(1R,2S,3R)-3-hydroxy-2-pentyl-1-cyclopentaneacétique et l'acide (-)-(1R,2S,3S)-3-hydroxy-2-pentyl-1-cyclopentaneacétique;
c) le traitement thermique dudit mélange dans le toluène, avec séparation de l'eau par distillation azéotropique, suivi d'un traitement avec carbonate de potassium pour isoler la (-)-(1R,8S)-8-pentyl-2-oxabicyclo [3.2.1]octan-3-one formée; et
d) le traitement de cette lactone avec NaOH, suivi d'acidification, pour obtenir l'acide (+)-(1R,2S,3R)-3-hydroxy-2-pentyl-1-cyclopentane-acétique pratiquement pur;
e) l'oxydation de cet acide à l'aide de chlorochromate de pyridinium, en présence d'acétate de sodium; et, le cas échéant,
f) l'estérification, dans des conditions appropriées, de l'acide (+)-(1R)-cis-3-oxo-2-pentyl-1-cyclopentaneacétique ainsi formé, pour obtenir le (+)-(1R)-cis-3-oxo-2-pentyl-1-cyclopentaneacétate de méthyle pratiquement pur.

11. Procédé selon la revendication 10, caractérisé en ce qu'on traite l'acide (+)-(1R,2S,3R)-3-hydroxy-2-pentyl-1-cyclopentaneacétique pratiquement pur obtenu à l'étape d), avant son oxydation, avec une amine de formule 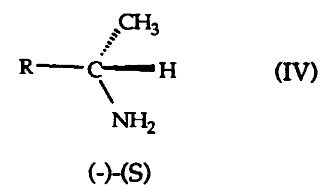 dans laquelle R représente un radical phényle ou 1-naphtyle, on cristallise le sel formé dans un solvant organique approprié et on acidifie ensuite le sel cristallin ainsi obtenu pour obtenir l'acide susmentionnée avec une pureté d'au moins 95%.

12. Procédé selon l'une des revendications précédentes, caractérisé en ce que l'estérification de l'acide (+)-(1R)-cis-3-oxo-2-pentyl-1-cyclopentane acétique est effectuée à l'aide de
a. dicarbonate de diméthyle dans le méthanol; ou
b. un mélange de sulfate de diméthyle et carbonate de potassium, dans un solvant approprié.

13. Procédé pour la préparation de l'acide (+)-(1R)-cis-3-oxo-2-pentyl-1-cyclopentaneacétique ou du (+)-(1R)-cis-3-oxo-2-pentyl-1-cyclopentaneacétate de méthyle, ayant une pureté d'au moins 95%, caractérisé en ce qu'on oxyde l'acide (+)-(1R,2S,3R)-3-hydroxy-2-pentyl-1-cyclopentaneacétique à l'aide de chlorochromate de pyridinium, en présence d'acétate de sodium, et, le cas échéant, on estérifie dans des conditions appropriées l'acide (+)-(1R)-cis-3-oxo-2-pentyl-1-cyclopentaneacétique ainsi formé.

14. Procédé selon la revendication 13, caractérisé en ce que l'acide (+)-(1R,2S,3R)-3-hydroxy-2-pentyl-1-cyclopentaneacétique de départ est préparé par traitement de l'acide c-3-hydroxy-c-2-pentyl-r-1-cyclopentaneacétique avec une amine de formule 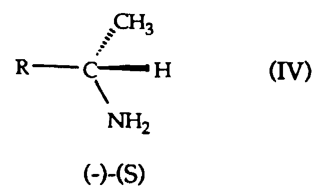 dans laquelle R représente un radical phényle ou 1-naphtyle, suivi de cristallisation du sel ainsi formé dans un solvant organique approprié et acidification du sel cristallin obtenu.

15. Procédé selon la revendication 14, caractérisé en ce que l'acide c-3-hydroxy-c-2-pentyl-r-1-cyclopentaneacétique est préparé selon une méthode qui consiste en:
a) la réduction du 3-oxo-2-pentyl-1-cydopentaneacétate de méthyle à l'aide de tri-(sec-butyl)-borohydrure de lithium ;
b) le traitement du produit de la réaction avec H₂O₂ et un excès de NaOH, suivi d'acidification, pour obtenir un mélange d'acides c-3-hydroxy-c-2-pentyl-r-1-cydopentaneacétique et t-3-hydroxy-t-2-pentyl-r-1-cyclopentane acétique;
c) le traitement thermique dudit mélange dans le toluène, avec séparation de l'eau par distillation azéotropique, suivi d'un traitement avec carbonate de potassium pour isoler la (1RS,8SR)-8-pentyl-2-oxabicyclo [3.2.1]octan-3-one formée; et
d) le traitement de cette lactone avec NaOH, suivi d'acidification.

16. Procédé selon la revendication 13, caractérisé en ce qu'on estérifie l'acide (+)-(1R)-cis-3-oxo-2-pentyl-1-cyclopentaneacétique à l'aide de
a. dicarbonate de diméthyle dans le méthanol; ou
b. un mélange de sulfate de diméthyle et carbonate de potassium, dans un solvant approprié.

17. Composé de formule 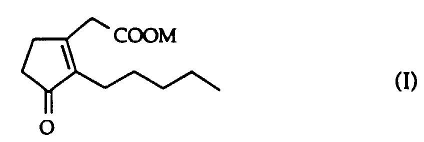 dans laquelle le symbole M représente un atome d'un métal alcalin ou alcalino-terreux ou un groupe NR₄, R représentant l'hydrogène ou un radical alkyle inférieur.

18. Composé sélectionné dans le groupe constitué par:
a. acide (+)-(1R)-cis-3-oxo-2-pentyl-1-cyclopentaneacétique ayant une pureté stéréochimique supérieure à 95% et un excès énantiomérique d'au moins 90%;
b. acide (+)-(1R)-cis-3-oxo-2-pentyl-1-cyclopentaneacétique caractérisé par un [α]²⁰_{D} supérieur à +71° (c = 0,985 g/100 ml, éthanol);
c. (+)-(1R)-cis-3-oxo-2-pentyl-1-cyclopentaneacétate de méthyle ayant une pureté stéréochimique supérieure à 95% et un excès énantiomérique d'au moins 90%;
d. (+)-(1R)-cis-3-oxo-2-pentyl-1-cyclopentaneacétate de méthyle caractérisé par un [α]²⁰_{D} supérieur à +69° (c = 1,49 g/100 ml, méthanol) ou à +83,5° (c = 1,19 g/100 ml, chloroforme);
e. acide (+)-(1R,2S,3R)-3-hydroxy-2-pentyl-1-cyclopentaneacétique caractérisé par un [α]²⁰_{D} supérieur à +11° (c = 1,10 g/100 ml, éthanol); et
f. (+)-(1R,2S,3R)-3-hydroxy-2-pentyl-1-cyclopentaneacétate de méthyle caractérisé par un [α]²⁰_{D} supérieur à +17° (c = 1,795 g/100 ml, méthanol).

## Patentansprüche

1. Verfahren zur Herstellung der 3-Oxo-2-pentyl-1-cyclopentanessigsäure oder des Methyl 3-oxo-2-pentyl-cyclopentanacetats, welche sich im wesentlichen in Form ihrer Isomeren der Konfiguration (+)-(1R)-cis befinden, dadurch gekennzeichnet, dass es die folgenden Stufen umfasst:
a) die katalytische Hydrierung einer Verbindung der Formel 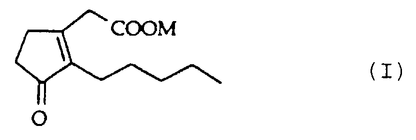 worin das Symbol M ein Wasserstoffatom, ein Alkali- oder Erdalkalimetallatom bedeutet oder für eine NR₄-Gruppe steht, wobei R Wasserstoff oder eine niedere Alkylgruppe bedeutet, in einem geeigneten Lösungsmittel und in Anwesenheit eines Katalysators, der aus einem Ru(II)-Komplex mit chiralen Liganden besteht, die einen Rest des Typus 2,2'-Bis (diphenylphosphino)-1,1'-binaphthyl (BINAP) oder des Typus 1,2-Bis(2,5-dialkylphospholano)benzenyl (DuPHOS) enthalten, wobei Alkyl einen C₂- oder C₃-Rest bedeutet, um eine Verbindung der Formel 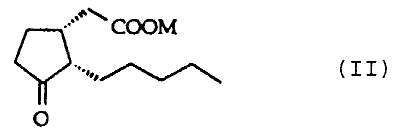 worin M die oben angegebene Bedeutung besitzt, welche sich im wesentlichen in Form des Isomeren der Konfiguration (+)-(1R)-cis befindet, zu erhalten;
b) gegebenenfalls die Ansäuerung der Verbindung der Formel (II) in an sich bekannter Weise, worin M ein Alkali- oder Erdalkalimetallatom bedeutet oder für eine NR₄-Gruppe steht, wobei R Wasserstoff oder einen niederen Alkylrest bedeutet, um die (+)-(1R)-cis-3-Oxo-2-pentyl-1-cyclopentanessigsäure zu erhalten; und
c) gegebenenfalls die unter geeigneten Bedingungen durchgeführte Veresterung der so erhaltenen (+)-(1R)-cis-3-Oxo-2-pentyl-1-cyclopentanessigsäure, um das (+)-(1R)-cis-Methyl 3-oxo-2-pentyl-1-cyclopentanacetat zu bilden.

2. Verfahren gemäss Patentanspruch 1, dadurch gekennzeichnet, dass man die 3-Oxo-2-pentyl-1-cyclopenten-1-essigsäure einer katalytischen Hydrierung in einem organischen Lösungsmittel in Anwesenheit von Bis(trifluoracetato)[(+)(R)-BINAP]Ruthenium(II) oder Bis(trifluoracetato)[(-)-(R,R)-Et-DuPHOS]Ruthenium(II) unterwirft.

3. Verfahren gemäss Patentanspruch 2, dadurch gekennzeichnet, dass die katalytische Hydrierung in Anwesenheit einer organischen Stickstoffbase erfolgt.

4. Verfahren gemäss Patentanspruch 3, dadurch gekennzeichnet, dass die organische Base Triethylamin ist.

5. Verfahren gemäss Patentanspruch 4, dadurch gekennzeichnet, dass das Triethylamin in einem Molverhältnis von 2:1 bis 4:1, bezogen auf den Ru(II)-Katalysator, verwendet wird.

6. Verfahren gemäss einem der Patentansprüche 2 bis 5, dadurch gekennzeichnet, dass das organische Lösungsmittel eine Mischung von Dichlormethan oder Tetrahydrofuran und Methanol ist.

7. Verfahren gemäss Patentanspruch 1, dadurch gekennzeichnet, dass man eine Verbindung der Formel (I), worin M für ein Alkali- oder Erdalkalimetallatom steht oder eine NR₄-Gruppe bedeutet, wobei R für Wasserstoff oder einen niederen Alkylrest steht, in einem geeigneten Lösungsmittel und in Anwesenheit eines Katalysators, welcher aus einem Komplex von Ruthenium(II) und (+)-(R)-BINAP-sulfoniert besteht, einer katalytischen Hydrierung unterwirft.

8. Verfahren gemäss Patentanspruch 7, dadurch gekennzeichnet, dass die Verbindung der Formel (I) das Lithium-3-oxo-2-pentyl-1-cyclopenten-1-acetat und der Katalysator das Bis(trifluoracetato) [(+)-(R)-BINAP-sulfoniert]Ruthenium(II) sind.

9. Verfahren gemäs Patentanspruch 7 oder 8, dadurch gekennzeichnet, dass das Lösungsmittel Wasser ist oder eine Mischung von Wasser und Methanol.

10. Verfahren gemäss einem der vorhergehenden Patentansprüche, dadurch gekennzeichnet, dass es zusätzlich die Reinigung der erhaltenen (+)-(1R) -cis-3-Oxo-2-pentyl-1-cyclopentanessigsäure oder des (+)-(1R)-cis-Methyl 3-oxo-2-pentyl-1-cyclopentanacetats beinhaltet, wobei die Reinigung umfasst:
a) die Reduktion von (+)-(1R)-cis-Methyl 3-oxo-2-pentyl-1-cyclopentanacetat mit Hilfe von Lithium-tri- (sec. butyl)-borhydrid;
b) die Behandlung des so erhaltenen Reaktionsgemisches mit H₂O₂ und einem Überschuss von NaOH und die nachfolgende Ansäuerung des erhaltenen Salzes, um eine Mischung zu erhalten, welche im wesentlichen die (+)-(1R,2S,3R)-3-Hydroxy-2-pentyl-1-cyclopentanessigsäure und die (-)-(1R,2S,3S)-3-Hydroxy-2-pentyl-1-cyclopentanessigsäure enthält;
c) die thermische Behandlung dieser Mischung in Toluol, mit Abtrennung von Wasser mittels azeotroper Destillation, gefolgt von einer Behandlung mit Kaliumcarbonat, um das gebildete (-)-(1R,8S)-8-Pentyl-2-oxabicyclo[3,2,1]octan-3-on zu isolieren; und
d) die Behandlung dieses Laktons mit NaOH, gefolgt von einer Ansäuerung, um die praktisch reine (+)-(1R,2S,3R)-3-Hydroxy-2-pentyl-1-cyclopentanessigsäure zu erhalten;
e) die Oxydation dieser Säure mit Hilfe von Pyridinium-chlor-chromat in Anwesenheit von Natriumacetat; und gegebenenfalls
f) die Veresterung der so gebildeten (+)-(1R)-cis-3-Oxo-2-pentyl-1-cyclopentanessigsäure unter geeigneten Bedingungen, um das praktisch reine (+)-(1R)-cis-Methyl 3-oxo-2-pentyl-1-cyclopentanacetat zu erhalten.

11. Verfahren gemäss Patentanspruch 10, dadurch gekennzeichnet, dass man die in der Stufe d) erhaltene, praktisch reine (+)-(1R,2S,3R)-3-Hydroxy-2-pentyl-1-cyclopentanessigsäure vor ihrer Oxydation mit einem Amin der Formel 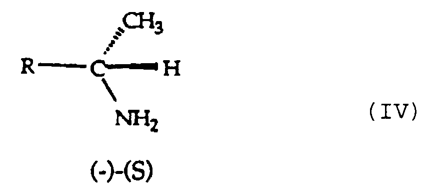 worin R für einen Phenyl- oder 1-Naphthylrest steht, behandelt, das gebildete Salz aus einem geeigneten organischen Lösungsmittel kristallisiert und anschliessend das so erhaltene kristalline Salz ansäuert, um die oben erwähnte Säure mit einer Reinheit von mindestens 95% zu erhalten.

12. Verfahren gemäss einem der vorhergehenden Patentansprüche, dadurch gekennzeichnet, dass die Veresterung der (+)-(1R)-cis-3-Oxo-2-pentyl-1-cyclopentanessigsäure mit Hilfe von:
a. Dimethyldicarbonat in Methanol, oder
b. einer Mischung von Dimethylsulfat und Kaliumcarbonat in einem geeigneten Lösungsmittel durchgeführt wird.

13. Verfahren zur Herstellung der (+)-(1R)-cis-3-Oxo-2-pentyl-1-cyclopentanessigsäure oder des (+)-(1R)-cis-Methyl 3-oxo-2-pentyl-1-cyclopentanacetats, welche eine Reinheit von mindestens 95% besitzen, dadurch gekennzeichnet, dass man die (+)-(1R,2S,3R)-3-Hydroxy-2-pentyl-1-cyclopentanessigsäure mit Hilfe von Pyridiniumchlorchromat in Anwesenheit von Natriumacetat oxydiert und gegebenenfalls die so gebildete (+)-(1R)-cis-3-Oxo-2-pentyl-1-cyclopentanessigsäure unter geeigneten Bedingungen verestert.

14. Verfahren gemäss Patentanspruch 13, dadurch gekennzeichnet, dass die als Ausgangsverbindung verwendete (+)-(1R,2S,3R)-3-Hydroxy-2-pentyl-1-cyclopentanessigsäure durch Behandlung der c-3-Hydroxy-c-2-pentyl-r-1-cyclopentanessigsäure mit einem Amin der Formel 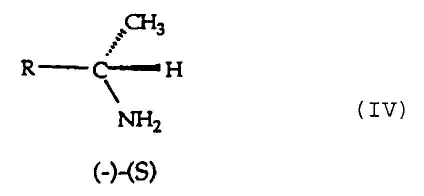 worin R für einen Phenyl- oder 1-Naphthylrest steht, gefolgt von einer Kristallisation des so gebildeten Salzes aus einem geeigneten organischen Lösungsmittel und Ansäuerung des erhaltenen kristallinen Salzes, hergestellt wird.

15. Verfahren gemäss Patentanspruch 14, dadurch gekennzeichnet, dass die c-3-Hydroxy-c-2-pentyl-r-1-cyclopentanessigsäure gemäss einem Verfahren hergestellt wird, das aus
a) der Reduktion von Methyl 3-oxo-2-pentyl-1-cyclopentanacetat mit Hilfe von Lithium-tri-(sec. butyl)-borhydrid;
b) der Behandlung des Reaktionsproduktes mit H₂O₂ und einem Überschuss von NaOH, gefolgt von einer Ansäuerung, um eine Mischung der c-3-Hydroxy-c-2-pentyl-r-1-cyclopentanessigsäure und der t-3-Hydroxy-t-2-pentyl-r-1-cyclopentanessigsäure zu erhalten;
c) der thermischen Behandlung dieser Mischung in Toluol, mit Abtrennung von Wasser mittels azeotroper Destillation, gefolgt von einer Behandlung mit Kaliumcarbonat, um das gebildete (1RS,8SR)-8-Pentyl-2-oxabicyclo[3,2,1]octan-3-on zu isolieren; und
d) der Behandlung dieses Laktons mit NaOH, gefolgt von einer Ansäuerung, besteht.

16. Verfahren gemäss Patentanspruch 13, dadurch gekennzeichnet, dass man die (+)-(1R)-cis-3-Oxo-2-pentyl-l-cyclopentanessigsäure mit Hilfe von
a. Dimethyldicarbonat in Methanol; oder
b. einer Mischung von Dimethylsulfat und Kaliumcarbonat in einem geeigneten Lösungsmittel verestert.

17. Verbindung der Formel 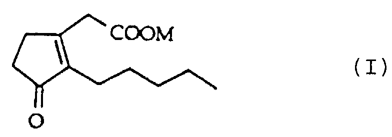 worin das Symbol M ein Alkali- oder Erdalkalimetallatom bedeutet oder für eine NR₄-Gruppe steht, wobei R Wasserstoff oder einen niederen Alkylrest bedeutet.

18. Verbindung ausgewählt aus der Gruppe bestehend aus:
a. (+)-(1R)-cis-3-Oxo-2-pentyl-1-cyclopentanessigsäuremit einer stereochemischen Reinheit von mehr als 95% und einem Enantiomerenüberschuss von mindestens 90%;
b. (+)-(1R)-cis-3-Oxo-2-pentyl-1-cyclopentanessigsäure, gekennzeichnet durch ein [α]²⁰_{D} von mehr als +71° (c = 0,985 g/100 ml, Ethanol);
c. (+)-(1R)-cis-Methyl 3-oxo-2-pentyl-1-cyclopentanacetat mit einer stereochemischen Reinheit von mehr als 95% und einem Enantiomerenüberschuss von mindestens 90%;
d. (+)-(1R)-cis-Methyl 3-oxo-2-pentyl-1-cyclopentanacetat, gekennzeichnet durch ein [α]²⁰_{D} von mehr als +69° (c = 1,49 g/100 ml, Methanol) oder mehr als +83,5° (c = 1,19 g/100 ml, Chloroform);
e. (+)-(1R,2S,3R)-3-Hydroxy-2-pentyl-1-cyclopentanessigsäure, gekennzeichnet durch ein [α]²⁰_{D} von mehr als +11° (c = 1,10 g/100 ml, Ethanol); und
f. (+)-(1R,2S,3R)-Methyl 3-hydroxy-2-pentyl-1-cyclopentanacetat, gekennzeichnet durch ein [α]²⁰_{D} von mehr als +17°(c = 1,795 g/100 ml, Methanol).

## Claims

1. Process for the preparation of 3-oxo-2-pentyl-1-cyclopentaneacetic acid, or of methyl 3-oxo-2-pentyl-1-cyclopentaneacetate, essentially in the form of their (+)-(1R)-cis configuration isomer, characterized in that it comprises the following steps :
a) the catalytic hydrogenation, in an appropriate solvent and in the presence of a catalyst composed of a Ru (II) complex comprising chiral ligands which contain a 2,2'-bis(diphenylphosphino)-1,1'-binaphthyl (BINAP) type ligand or a 1,2-bis(2,5-dialkylphospholano)benzenyl (DuPHOS) type ligand, wherein the alkyl is a C₂ or C₃ radical, of a compound of formula 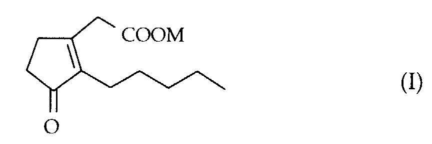 wherein M represents a hydrogen atom, an atom of an alkaline or alkaline-earth metal, or a NR₄ group, R representing hydrogen or a lower alkyl radical, to obtain a compound of formula 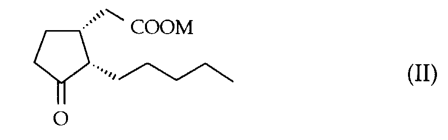 wherein M has the meaning indicated above, essentially in the form of the isomer of (+)-(1R)-cis configuration;
b) if necessary, the acidification, in a generally known manner, of the compound of formula (II) wherein M represents an atom of an alkaline or alkaline-earth metal or a NR₄ group, R representing hydrogen or a lower alkyl radical, to obtain (+)-(lR)-cis-3-oxo-2-pentyl-1-cyclopentaneacetic acid ; and
c) if necessary, the esterification, under appropriate conditions, of the (+)-(1R)-cis-3-oxo-2-pentyl-1-cyclopentaneacetic acid thus obtained to form methyl (+)-(1R)-cis-3-oxo-2-pentyl-1-cyclopentaneacetate.

2. Process according to claim 1, characterized in that 3-oxo-2-pentyl-1-cyclopentene-1-acetic acetic is subjected to catalytic hydrogenation in an organic solvent, in the presence of bis(trifluoroacetato)[(+)-(R)-BINAP]ruthenium (II) or bis(trifluoroacetato)[(-)-(R,R)-Et-DuPHOS] ruthenium (II).

3. Process according to claim 2, characterized in that the hydrogenation takes place in the presence of a nitrogen-containing organic base.

4. Process according to claim 3, characterized in that the organic base is triethylamine.

5. Process according to claim 4, characterized in that triethylamine is used in a molar ratio of 2:1 to 4:1, relative to the Ru (II) catalyst.

6. Process according to any one of claims 2 to 5, characterized in that the organic solvent is a mixture of dichloromethane or tetrahydrofuran and methanol.

7. Process according to claim 1, characterized in that there is subjected to catalytic hydrogenation, in an appropriate solvent and in the presence of a catalyst composed of a ruthenium (II) complex and (+)-(R)-BINAP-sulfonated, a compound of formula (I) wherein M represents an atom of an alkaline or alkaline-earth metal or a NR₄ group, R representing hydrogen or a lower alkyl radical.

8. Process according to claim 7, characterized in that the compound of formula (I) is lithium 3-oxo-2-pentyl-1-cyclopentene-1-acetate and the catalyst is bis(trifluoroacetato) [(+)-(R)-BINAP-sulfonated]ruthenium (II).

9. Process according to claim 7 or 8, characterized in that the solvent is water or a mixture of water and methanol.

10. Process according to any one of the preceding claims, characterized in that it further comprises the purification of the obtained (+)-(1R)-cis-3-oxo-2-pentyl-1-cyclopentaneacetic acid or of methyl (+)-(1R)-cis-3-oxo-2-pentyl-1-cyclo-pentaneacetate, which purification comprises :
a) the reduction of methyl (+)-(1R)-cis-3-oxo-2-pentyl-1-cyclopentane-acetate by means of lithium tri-(sec-butyl)-borohydride;
b) the treatment of the thus obtained reaction mixture with H₂O₂ and an excess of NaOH, followed by the acidification of the resulting salt, to obtain a mixture which contains essentially (+)-(1R,2S,3R)-3-hydroxy-2-pentyl-1-cyclopentaneacetic acid and (-)-(1R,2S,3S)-3-hydroxy-2-pentyl-1-cyclopentaneacetic acid ;
c) the thermal treatment of said mixture in toluene, with water separation via azeotropic distillation, followed by a treatment with potassium carbonate to isolate the formed (-)-(1R,8S)-8-pentyl-2-oxabicyclo [3.2.1]octan-3-one; and
d) the treatment of this lactone with NaOH, followed by acidification, to obtain practically pure (+)-(1R,2S,3R)-3-hydroxy-2-pentyl-1-cyclopentaneacetic acid ;
e) the oxidation of this acid by means of pyridinium chlorochromate, in the presence of sodium acetate; and, if necessary,
f) the esterification, under appropriate conditions, of the thus formed (+)-(1R)-cis-3-oxo-2-pentyl-1-cyclopentaneacetic acid, to obtain practically pure methyl (+)-(1R)-cis-3-oxo-2-pentyl-1-cyclopentaneacetate.

11. Process according to claim 10, characterized in that the pratically pure (+)-(1R,2S,3R)-3-hydroxy-2-pentyl-1-cyclopentaneacetic acid obtained in step d) is treated, before its oxidation, with an amine of formula 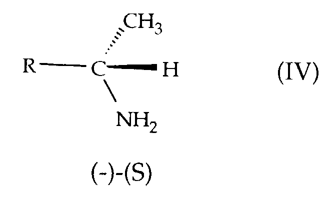 wherein R represents a phenyl or 1-naphthyl radical, the resulting salt is crystallized in an appropriate organic solvent and the thus obtained crystalline salt is then acidified to obtain the above-mentioned acid with at least 95% purity.

12. Process according to any one of the preceding claims, characterized in that the esterification of (+)-(1R)-cis-3-oxo-2-pentyl-1-cyclopentaneacetic acid is carried out by means of
a. dimethyl dicarbonate in methanol ; or
b. a mixture of dimethyl sulfate and potassium carbonate, in an appropriate solvent.

13. Process for the preparation of (+)-(1R)-cis-3-oxo-2-pentyl-1-cyclopentaneacetic acid or of methyl (+)-(1R)-cis-3-oxo-2-pentyl-1-cyclopentaneacetate, having a purity of at least 95%, characterized in that (+)-(1R,2S,3R)-3-hydroxy-2-pentyl-1-cyclopentaneacetic acid is oxidized by means of pyridinium chlorochromate, in the presence of sodium acetate, and, if necessary, the thus formed (+)-(1R)-cis-3-oxo-2-pentyl-1-cyclopentaneacetic acid is esterified under appropriate conditions.

14. Process according to claim 13, characterized in that the starting (+)-(1R,2S,3R)-3-hydroxy-2-pentyl-1-cyclopentaneacetic acid is prepared by treating c-3-hydroxy-c-2-pentyl-r-1-cyclopentaneacetic acid with an amine of formula 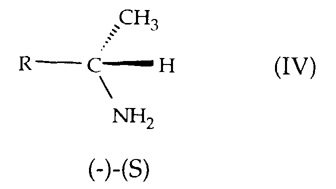 wherein R represents a phenyl or 1-naphthyl radical, followed by crystallization of the thus formed salt in an appropriate organic solvent and acidification of the obtained crystalline salt.

15. Process according to claim 14, characterized in that the c-3-hydroxy-c-2-pentyl-r-1-cyclopentaneacetic acid is prepared according to a method which consists in :
a) reducing methyl 3-oxo-2-pentyl-1-cyclopentaneacetate by means of lithium tri-(sec-butyl)-borohydride;
b) treating the reaction product with H₂O₂ and an excess of NaOH, followed by acidification, to obtain a mixture of c-3-hydroxy-c-2-pentyl-r-1-cyclopentaneacetic and t-3-hydroxy-t-2-pentyl-r-1-cyclopentaneacetic acids ;
c) thermally treating said mixture in toluene, with water separation by azeotropic distillation, followed by a treatment with potassium carbonate to isolate the formed (1RS,8SR)-8-pentyl-2-oxabicyclo [3.2.1]octan-3-one; and
d) treating this lactone with NaOH, followed by acidification.

16. Process according to claim 13, characterized in that (+)-(1R)-cis-3-oxo-2-pentyl-1-cyclopentaneacetic acid is esterified by means of
a. dimethyl dicarbonate in methanol; or
b. a mixture of dimethyl sulfate and potassium carbonate, in an appropriate solvent.

17. Compound of formula 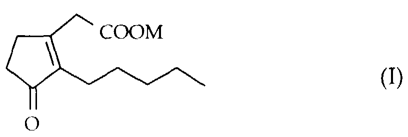 wherein symbol M represents an atom of an alkaline or alkaline-earth metal, or a NR₄ group, R representing hydrogen or a lower alkyl radical.

18. Compound selected from the group consisting of :
a. (+)-(1R)-cis-3-oxo-2-pentyl-1-cyclopentaneacetic acid having a stereochemical purity above 95% and an enantiomeric excess of at least 90%;
b. (+)-(1R)-cis-3-oxo-2-pentyl-1-cyclopentaneacetic acid characterized by a [α]₂₀^{D} above +71° (c = 0.985 g/100 ml, ethanol);
c. methyl (+)-(1R)-cis-3-oxo-2-pentyl-1-cyclopentaneacetate having a stereochemical purity above 95% and an enatiomeric excess of at least 90%;
d. methyl (+)-(1R)-cis-3-oxo-2-pentyl-1-cyclopentaneacetate characterized by a [α]₂₀^{D} above +69° (c = 1.49 g/100 ml, methanol) or above +83.5° (c = 1.19 g/100 ml, chloroform);
e. (+)-(1R,2S,3R)-3-hydroxy-2-pentyl-1-cyclopentaneacetic acid, characterized by a [α]₂₀^{D} above +11° (c = 1.10 g/100 ml, ethanol); and
f methyl (+)-(1R,2S,3R)-3-hydroxy-2-pentyl-1-cyclopentaneacetate, characterized by a [α]₂₀^{D} above +17° (c = 1.795 g/100 ml, methanol).
